# EUROPÄISCHE PATENTSCHRIFT

(11) **EP 1 648 531 B1**
(45) Veröffentlichungstag und Bekanntmachung des Hinweises auf die Patenterteilung: **01.10.2008**
(21) Anmeldenummer: 04762421.8
(22) Anmeldetag: 16.07.2004
(51) Int. Cl.: A61L 24/06

(54) **VERWENDUNG VON ANTISEPTISCHEN WIRKSTOFFEN IN PMMA-KNOCHENZEMENTEN**
USE OF ANTISEPTIC ACTIVE PRINCIPLES IN PMMA BONE CEMENTS
UTILISATION DE PRINCIPES ACTIFS ANTISEPTIQUES DANS DES CIMENTS OSSEUX EN PMMA

(30) Priorität: 18.07.2003 DE 10332680
(43) Veröffentlichungstag der Anmeldung: 26.04.2006
(73) Patentinhaber: Biomet Deutschland GmbH, 14167 Berlin (DE)
(72) Erfinder: SCHILKE, Frank, 64331 Weiterstadt (DE); NIES, Berthold, 64407 Fraenkisch-Crumbach (DE); PIETSCH, Hanns, 20148 Hamburg (DE)
(74) Vertreter: Gross, Felix
(86) Internationale Anmeldenummer: PCT/DE2004/001571
(87) Internationale Veröffentlichungsnummer: WO 2005/009495

(56) Entgegenhaltungen:
- EP-A- 0 450 117
- EP-A- 0 985 413
- WO-A-01/12242
- US-A- 4 797 282
- US-A- 5 942 218
- US-A1- 2002 041 899
- US-B1- 6 494 717

## Beschreibung

Die Erfindung betrifft die Verwendung von antiseptischen Wirkstoffen in Polymethylmethacrylat-Knochenzementen (PMMA-Zement) mit einer zur Verhinderung der mikrobiellen Besiedlung der Zementoberfläche ausreichenden Wirkstoffkonzentration.

Herkömmliche arzneimittelhaltige Knochenzemente bestehen aus einem PMMA- bzw. PMMA-Copolymer-Pulver, in welchem u.a. das pulverförmige Arzneimittel verteilt ist. Nach dem Zumischen einer Monomerflüssigkeit (mit einem Aktivator) kommt es zur Polymerisation. Der ausgehärtete Knochenzement ist danach eine Polymermasse, aus der das in der Oberflächenschicht befindliche Arzneimittel freigesetzt wird.

Zur Verhinderung septischer Entzündungsreaktionen nach mikrobieller Besiedelung des Zementes und/oder des angrenzenden Gewebes werden im herkömmlichen Knochenzement Antibiotika als Arzneimittel verwendet. Der weit verbreitete Einsatz von Antibiotika in Knochenzementen führt jedoch in zunehmendem Maße zur Ausbildung antibiotikaresistenter Bakterienstämme, wodurch Wundinfektionen u.U. nicht mehr vollständig verhindert werden können. Auch der Einsatz neuerer Antibiotika stellt keine dauerhafte Lösung dar, da sich in absehbarer Zeit gegen das neue Arzneimittel resistente Bakterienstämme bilden werden.

Die EP 701 824 (Merck Patent GmbH) beschreibt ein Verfahren zur Herstellung von wirkstoffhaltigen Knochenzementen, die u.a. auch Antibiotika oder Antiseptika enthalten können.

Die WO 98/07456 (Merck Patent GmbH) betrifft ein Verfahren zur Herstellung von wirkstoffhaltigen Knochenzementen, sowie daraus hergestellter Knochenersatzmaterialien oder implantierbarer Pharmakadepots, die u.a. auch Antibiotika oder Antiseptika enthalten können.

Die EP 202 445 (Merck Patent GmbH) betrifft ein im Körper implantierbares Pharmakadepot zur kontrollierten verzögerten Freisetzung von Cytostatika, dass neben einem Cytostatikum noch ein Antibiotikum und/oder Antiseptikum enthalten kann.

Die EP 234 004 (Merck Patent GmbH) beschreibt ein implantierbares Pharmakadepot, welches Antibiotika und Antiseptika zur Steigerung oder Ergänzung der Wirkung des Chemotherapeutikums enthält.

Aufgabe der Erfindung ist es, das Antibiotikum in herkömmlichen Knochenzementen durch ein neues Arzneimittel zu ersetzen, ohne dass die antibakterielle Wirkung an der Oberfläche des Zementes negativ beeinträchtigt wird. Dabei soll das neue Arzneimittel aufgrund seines anderen Wirkmechanismus dauerhaft die Bildung resistenter Bakterien verhindern. Das neue Arzneimittel soll in Art und Konzentration so gewählt werden, dass die antibakterielle Wirkung gewährleistet ist, die Wundheilung aber nicht wesentlich beeinträchtigt wird.

Gelöst wird die Aufgabe durch die Verwendung von antiseptischen Wirkstoffen in einem PMMA-Knochenzement mit einer zur Verhinderung der mikrobiellen Besiedlung der Zementoberfläche ausreichenden Wirkstoffkonzentration. Vorzugsweise enthält der PMMA-Knochenzement kein Antibiotikum.

Als Antiseptika kommen Verbindungen aus den folgenden Gruppen in Betracht:
• Quaternäre Ammoniumverbindungen wie Hexadecyldimethylethylammoniumethosulfat oder Didecyldimethylammoniumchlorid,
• Aminoxide wie N-Alkyl(C10 - C18)-N,N-dimethylamin-N-oxid oder N-Alkyl-(C10- C18)- N,N-diethylamin-N-oxid,
• Pyridinderivate wie Octenidindihydrochlorid,
• Guanidine wie Polyhexamethylenbiguanidhydrochlorid, und/oder
• 10-Undecylensäureamide wie 10-Undecylensäure-N-ethanolamid.

Vorzugsweise wird Polyhexamethylenbiguanid in einer Menge von maximal 1 Masse-% bezogen auf die Gesamtmasse des Zementes zugesetzt. Noch bevorzugter ist eine Menge von maximal 0.5 Masse-%, wobei noch viel bevorzugter eine Menge von 0.025 bis 0.5 Masse-% ist. Am bevorzugtesten ist eine Menge von maximal 0.155 Masse-% Polyhexamethylenbiguanid. Es kann erfindungsgemäß auch mehr als ein antiseptischer Wirkstoff zugesetzt werden.

### Ergebnisse der Vergleichsversuche:

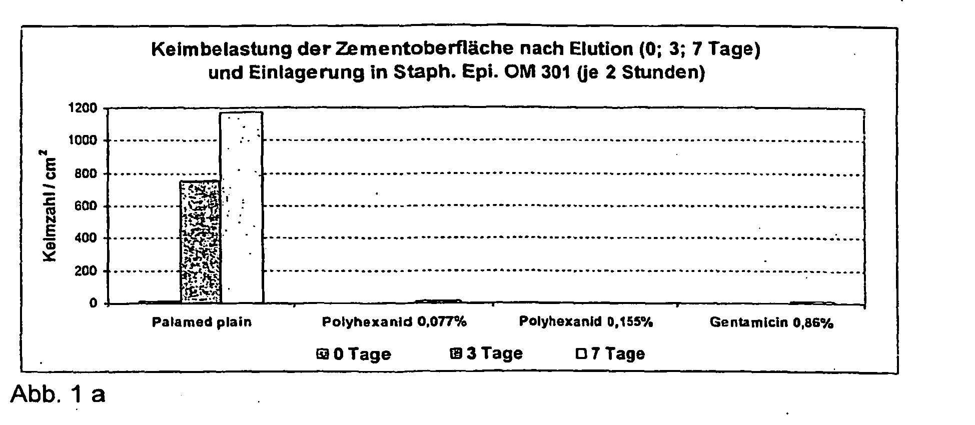

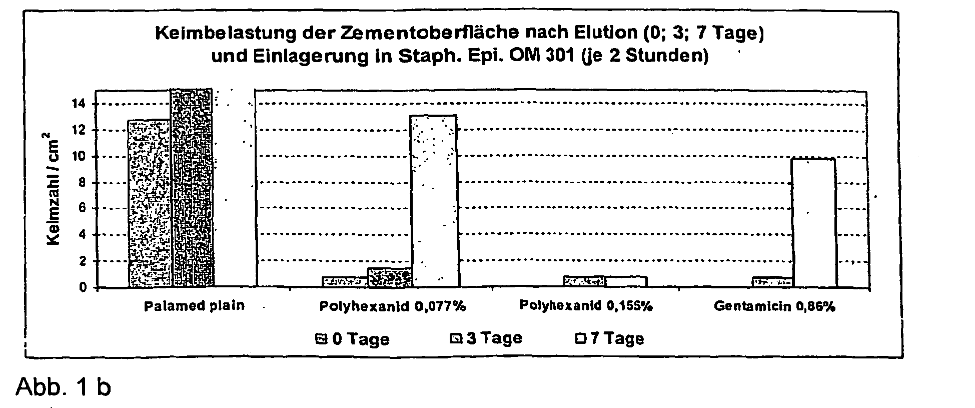

Wie aus den Abbildungen 1 a bzw. 1 b zu erkennen ist, hat eine Beimischung von nur 0.155 Masse-% Polyhexamethylenbiguanid zu einem PMMA-Knochenzement (PALAMED^{®} plain) die gleiche (oder eine höhere) biologische Wirksamkeit bei der Verhinderung der Besiedlung der Zementoberfläche mit Keimen wie die zum Vergleich herangezogene Beimischung von 0.86 Masse-% Gentamicin (Antibiotikum).

Die Herstellung des erfindungsmäßen Knochenzementes wird anhand zweier Beispiele genauer beschrieben.

### Beispiel 1:

In 18,8 g Palamed-Flüssigkeit (bestehend aus Methylmethacrylat, N,N-Dimethyl-p-toluidin und Farbstoff) wurden 97,3 mg Polyhexamethylenbiguanid-hydrochlorid eingemischt. Die homogene Lösung wurde mit 44 g Palamed-Pulver (plain; ohne Gentamicin) in einem Vakuum-Mixsystem nach Angaben des Herstellers vermischt. Die Mischung wurde in Formen gegeben und ausgehärtet.

### Beispiel 2:

5,3 g Zirkoniumdioxid wurden mit einer Lösung aus 97,3 mg Polyhexamethylenbiguanid-hydrochlorid in 400 mg Wasser vermengt. Das Wasser wurde durch Gefriertrocknung entfernt. Anschließend wurde das Antiseptikum-haltige Zirkoniumdioxid mit 38,3 g Poly(methylmethacrylat-coethylacrylat) und 0,44 g Dibenzoylperoxid vermischt. Das entstandene Pulver wurde zu einer Lösung von 0,4 g *N,N*-Dimethyl-p-toluidin in 18,4 g Methylmethacrylat gegeben und beides intensiv vermischt. Die Mischung wurde in Formen gegeben und ausgehärtet.

## Patentansprüche

1. Verwendung des Antiseptikums Polyhexamethylenbiguanid in PMMA-Knochenzementen mit einer zur Verhinderung der mikrobiellen Besiedlung der Zementoberfläche ausreichenden Wirkstoffkonzentration von maximal 1 Masse % bezogen auf die Gesamtmenge des Zementes.

2. Verwendung von Polyhexamethylenbiguanid nach Anspruch 1 in PMMA-Zementen, die kein Antibiotikum enthalten.

3. Verwendung nach einem der Ansprüche 1 oder 2 in einer Menge von 0,025 bis 0,5 Masse-% bezogen auf die Gesamtmenge des Zementes.

4. Verwendung nach Anspruch 3 in einer Menge von maximal 0.155 Masse-% bezogen auf die Gesamtmenge des Zementes.

5. Medizinische Implantate, hergestellt aus PMMA-Knochenzement enthaltend eine zur Verhinderung der mikrobiellen Besiedlung der Zementoberfläche ausreichenden Wirkstoffkonzentration von maximal 1 Masse% des Antiseptikums Polyhexamethylenbiguanid bezogen auf die Gesamtmenge des Zementes.

## Claims

1. The use of the antiseptic polyhexamethylene biguanide in PMMA bone cements at an active ingredient concentration, sufficient to prevent microbial colonisation of the cement surface, of not more than 1% by weight based on the total amount of the cement.

2. The use of polyhexamethylene biguanide,according to Claim 1 in PMMA cements which do not contain an antibiotic.

3. The use according to either of Claims 1 and 2 in an amount of 0.025% to 0.5% by weight based on the total amount of the cement.

4. The use according to Claim 3 in an amount of not more than 0.155% by weight based on the total amount of the cement.

5. Medical implants obtained from PMMA bone cement containing an active ingredient concentration, sufficient to prevent the microbial colonisation of the cement surface, of not more than 1% by weight of the antiseptic polyhexamethylene biguanide based on the total amount of the cement.

## Revendications

1. Utilisation de l'antiseptique polyhexaméthylènebiguanide dans des ciments à os à base de PMMA, à une concentration de substance active, suffisante pour empêcher la colonisation microbienne de la surface du ciment, d'au maximum 1 % en masse par rapport à la quantité totale du ciment.

2. Utilisation de polyhexaméthylènebiguanide selon la revendication 1, dans des ciments à base de PMMA qui ne contiennent pas d'antibiotique.

3. Utilisation selon une des revendications 1 ou 2, en une quantité de 0,025 à 0,5 % en masse, par rapport à la quantité totale du ciment.

4. Utilisation selon la revendication 3, en une quantité d'au maximum 0,155 % en masse, par rapport à la quantité totale du ciment.

5. Implants médicaux, fabriqués à partir de ciment à os à base de PPMA, contenant une concentration de substance active, suffisante pour empêcher la colonisation microbienne de la surface du ciment, d'au maximum 1 % en masse de l'antiseptique polyhexaméthylènebiguanide par rapport à la quantité totale du ciment.
